# EUROPÄISCHE PATENTANMELDUNG

(11) **EP 4 108 163 A1**
(43) Veröffentlichungstag der Anmeldung: **28.12.2022**
(21) Anmeldenummer: 22180059.2
(22) Anmeldetag: 21.06.2022
(51) Int. Cl.: A61B 5/00

(54) **3D KÖRPERSCANNER ZUM ERSTELLEN VON 3D KÖRPERMODELLEN**

(30) Priorität: 21.06.2021 DE 102021115999
(71) Anmelder: Naked Labs Austria GmbH, 1070 Wien (AT)
(72) Erfinder: SCHULTES, Gerhard, 1220 Wien (AT); KREUZGRUBER, Peter, 1070 Wien (AT)
(74) Vertreter: Canzler & Bergmeier Patentanwälte Partnerschaft mbB

(57) **Zusammenfassung**

Die Erfindung betrifft einen 3D Körperscanner (1) zum Erstellen von 3D Körpermodellen, insbesondere einer äußeren Körperform, einer Person (2) mit einer Scannereinheit (3), die zumindest einen Tiefensensor (4) zur räumlichen Erfassung eines Sichtfeldes (5) umfasst, und mit einer Plattform (6) zur Aufnahme der Person (2). Erfindungsgemäß umfasst der 3D Körperscanner (1) eine Energieübertragungsanordnung (10), mittels der elektrische Energie kontaktlos zwischen der Scannereinheit (3) und der Plattform (6) übertragen werden kann.

## Beschreibung

Die vorliegende Erfindung betrifft einen 3D Körperscanner zum Erstellen von 3D Körpermodellen, insbesondere einer äußeren Körperform, einer Person mit einer Scannereinheit, die zumindest einen Tiefensensor zur räumlichen Erfassung eines Sichtfeldes umfasst, und mit einer Plattform zur Aufnahme der Person.

Aus der DE 20 2009 017 401 U1 ist ein Erfassungsgerät zur Erfassung von Raumformen von Körpern bekannt. Nachteilig daran ist es, dass das Erfassungsgerät unflexibel in der Verwendung ist.

Aufgabe der vorliegenden Erfindung ist es, den Stand der Technik zu verbessern.

Die Aufgabe wird gelöst durch eine 3D Körperscanner mit den Merkmalen des unabhängigen Patentanspruchs.

Vorgeschlagen wird ein 3D Körperscanner zum Erstellen von 3D Körpermodellen einer Person. Bei dem 3D Körpermodell kann es sich um eine äußere Körperform, also beispielsweise eine Silhouette der Person, handeln. Der Einfachheit halber kann im Folgenden der Zusatz "3D" weggelassen werden. Es ist jedoch klar, dass es sich bei dem Körperscanner und bei dem Körpermodell um den 3D Körperscanner bzw. um das 3D Körpermodell handelt.

Der Körperscanner umfasst eine Scannereinheit, die zumindest einen Tiefensensor zur räumlichen Erfassung eines Sichtfeldes umfasst. Mit Hilfe der Scannereinheit bzw. mit Hilfe des Tiefensensors wird das Körpermodell erfasst bzw. ermittelt.

Des Weiteren umfasst der Körperscanner eine Plattform zur Aufnahme der Person. Die Plattform dient beispielsweise insbesondere dazu, um die Person an einen definierten Ort zu platzieren.

Erfindungsgemäß umfasst der Körperscanner eine Energieübertragungsanordnung, mittels der elektrische Energie kontaktlos zwischen der Scannereinheit und der Plattform übertragen werden kann. Dadurch kann die Plattform flexibel verwendet werden. Auf eine Verkabelung zur Energieübertragung zwischen der Scannereinheit und der Plattform kann dadurch verzichtet werden. Außerdem kann die Plattform infolgedessen flexibel platziert werden.

Vorteilhaft ist es, wenn die Plattform einen elektrischen Energiespeicher umfasst. Der elektrische Energiespeicher ist vorzugsweise mittels der Energieübertragungsanordnung aufladbar. Der elektrische Energiespeicher kann beispielsweise einen Akkumulator umfassen. Wenn der Körperscanner beispielsweise nicht verwendet wird, kann mittels der Energieübertragungsanordnung der Energiespeicher geladen werden. Wird der Körperscanner dann benutzt, wird die Plattform mit elektrischer Energie vom Energiespeicher versorgt. Zusätzlich oder alternativ kann auch Energie mittels der Energieübertragungsanordnung an die Plattform übertragen werden.

Von Vorteil ist es, wenn die Energieübertragungsanordnung eine Sendeeinheit zum Senden und eine Empfangseinheit zum Empfangen der elektrischen Energie umfasst. Dabei kann die Scannereinheit die Sendeeinheit und die Plattform die Empfangseinheit umfassen. Dadurch wird Energie von der Scannereinheit an die Plattform übertragen. Infolgedessen kann lediglich die Scannereinheit mit einem Stromnetz verbunden sein. Die Plattform ist dann vom Stromnetz getrennt. Dies weist ebenfalls einen Vorteil dahingehend auf, dass bei einer Überspannung im Stromnetz die Plattform nicht beschädigt werden kann.

Vorteilhaft ist es, wenn die Scannereinheit ein Fußelement aufweist, welches vorzugsweise die Sendeeinheit umfasst. Dadurch ist die Sendeeinheit bodennah, so dass die Energieübertragung zur Plattform effektiv ist.

Von Vorteil ist es, wenn die Scannereinheit, insbesondere das Fußelement, eine Aussparung aufweist und dass die Plattform einen zur Aussparung korrespondierenden Einführabschnitt aufweist, so dass die Plattform mittels dem Einführabschnitt in der Aussparung angeordnet werden kann. Dadurch können Fehler bei der Benutzung des Körperscanners vermieden werden. Wenn der Einführabschnitt in die Aussparung eingeführt ist, findet die Energieübertragung, beispielsweise zum Laden des Energiespeichers, statt. Eine falsche Orientierung der Plattform zur Scannereinheit, bei der keine Energieübertragung stattfinden kann, wird somit vermieden.

Vorteilhaft ist es, wenn die Scannereinheit, insbesondere das Fußelement, im Bereich der Aussparung die Sendeeinheit aufweist und die Plattform im Einführabschnitt die Empfangseinheit aufweist. Alternativ kann die Scannereinheit, insbesondere das Fußelement, im Bereich der Aussparung die Empfangseinheit und die Plattform im Einführabschnitt die Sendeeinheit aufweisen.

Von Vorteil ist es, wenn die Scannereinheit eine Koppelanordnung aufweist, so dass die Plattform zur Energieübertragung an die Scannereinheit angekoppelt werden kann. Zusätzlich oder alternativ kann die Plattform die Koppelanordnung aufweisen, so dass die Plattform zur Energieübertragung an die Scannereinheit angekoppelt werden kann. Auch dadurch wird die Sicherheit erhöht, dass die Energieübertragung stattfindet. Die Plattform und die Scannereinheit bleiben mittels der Koppelanordnung miteinander verbunden und können sich nicht voneinander lösen.

Vorteilhaft ist es, wenn zur Energieübertragung die Sendeeinheit und die Empfangseinheit deckungsgleich in einer Horizontalrichtung der Scannereinheit übereinander angeordnet werden können. Die Energieübertragung erfolgt somit in vertikaler Richtung und dies auf effektive Weise. Dadurch kann die Energieübertragungsanordnung platzsparend ausgebildet werden.

Von Vorteil ist es, wenn bei vorgesehener Platzierung des 3D Körperscanners die Sendeeinheit unter der Empfangseinheit angeordnet ist. Dadurch kann eine gute Effektivität bei der Energieübertragung erreicht werden.

Vorteilhaft ist es, wenn die Energieübertragungsanordnung derart ausgebildet ist, dass die elektrische Energie induktiv übertragen werden kann. Mit Hilfe der induktiven Energieübertragung können höhere Leistungen übertragen werden. Zusätzlich oder alternativ ist es vorteilhaft, wenn die Energieübertragungsanordnung derart ausgebildet ist, dass die elektrische Energie kapazitiv übertragen werden kann. Die kapazitive Energieübertragung ist von der Bauart einfacher auszubilden.

Von Vorteil ist es, wenn die Sendeeinheit eine Sendespule und die Empfangseinheit eine Empfangsspule umfasst, wobei zur Energieübertragung die Sendespule und die Empfangsspule koaxial zueinander angeordnet sind. Dadurch kann die induktive Energieübertragung ausgebildet werden. Zusätzlich oder alternativ kann die Sendeeinheit und die Empfangseinheit zur Ausbildung der kapazitiven Energieübertragung auch eine entsprechende Kondensatoranordnung aufweisen.

Vorteilhaft ist es, wenn der 3D Körperscanner zumindest einen 3D-Sensor umfasst. Der Tiefensensor kann beispielsweise als der 3D-Sensor ausgebildet sein. Zusätzlich oder alternativ kann der Tiefensensor und/oder der 3D-Sensor auch zumindest zwei Kameras aufweisen, um mittels Triangulation die Person erfassen zu können. Zusätzlich oder alternativ kann der Tiefensensor auch auf strukturiertem Licht, auf Lidar und/oder Radar basieren.

Von Vorteil ist es, wenn die Plattform eine Wiegeeinheit aufweist. Neben der Erfassung der Körperform der Person kann somit auch ein Gewicht der Person ermittelt werden. Zusätzlich oder alternativ kann die Plattform aber auch einen Körperfettsensor umfassen, mittels dem, beispielsweise mit Hilfe einer Widerstandsmessung des Körpers der Person, ein Körperfettanteil der Person gemessen werden kann. Weiterhin zusätzlich oder alternativ kann die Plattform aber auch einen Pulssensor oder andere Sensoren zum Ermitteln eines Körperzustandes aufweisen. Zusätzlich oder alternativ kann die Plattform auch als Drehteller ausgebildet sein. Um ein vollständiges 3D-Abbild der Person, also die vollständige 3D-Körperform, erfassen zu können, kann die Person mit Hilfe der Plattform als Drehteller gedreht werden, um alle Seiten der Person der Scannereinheit zuwenden zu können. Die Person kann somit weiter stillstehen. Die Plattform bzw. der Drehteller kann dabei einen Elektromotor umfassen, um die Person drehen zu können. Die Wiegeeinheit und/oder der Drehteller werden vorzugsweise mittels der Energieübertragungseinheit mit elektrischer Energie versorgt. Dabei kann auch erst der Energiespeicher der Plattform mit Hilfe der Energieübertragungseinheit geladen werden.

Vorteilhaft ist es auch, wenn die Scannereinheit und die Plattform jeweils eine Funkschnittstelle aufweisen, so dass zwischen der Scannereinheit und Plattform eine drahtlose Funkverbindung ausgebildet werden kann. Dadurch können zwischen der Plattform und der Scannereinheit Informationen und/oder Befehle übertragen werden. Beispielsweise kann damit eine Anweisung zum Drehen der Person übermittelt werden, wenn die Plattform als Drehteller ausgebildet ist. Zusätzlich oder alternativ können aber auch Gewichtsinformationen von der Wiegeeinheit oder dem Körperfettsensor an die Scannereinheit übertragen werden.

Von Vorteil ist es, wenn die Scannereinheit einen Spiegel umfasst. Dadurch kann sich die Person selbst betrachten.

Vorteilhaft ist es ferner, wenn der Körperscanner eine Steuereinheit aufweist, welche die Energieübertragung, die Erfassung der Person, die Datenübertragung zwischen Plattform und Scannereinheit und/oder die Auswertung der erfassten Daten ausführt. Insbesondere kann die Steuereinheit anhand der erfassten Daten mittels des Tiefensensors auch die Körperform der Person ermitteln.

Weitere Vorteile der Erfindung sind in den nachfolgenden Ausführungsbeispielen beschrieben. Es zeigen:
- **Figur 1**: eine schematische Darstellung eines Körperscanners mit der Person und
- **Figur 2**: eine schematische Schnittansicht der an die Scannereinheit an-gekoppelten Plattform mit einer Energieübertragungsanordnung.

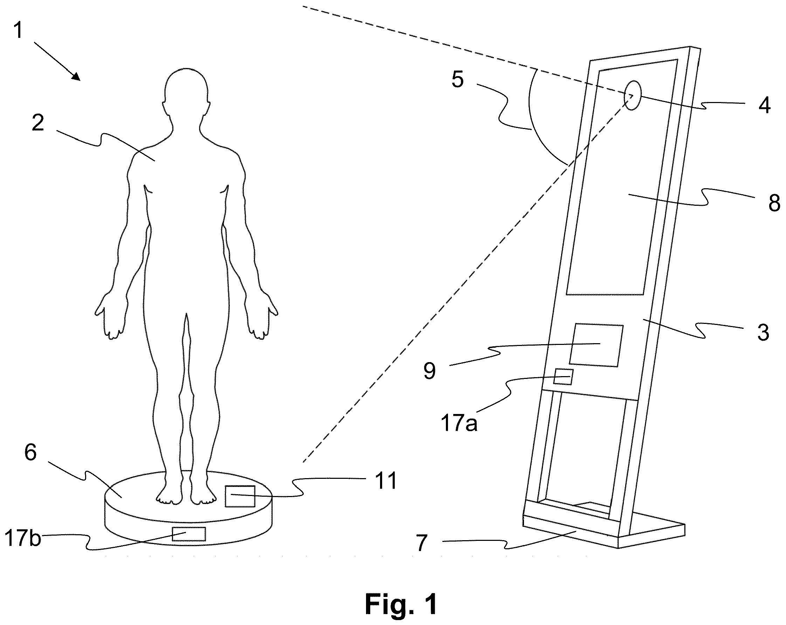

Figur 1 zeigt eine schematische Darstellung eines Körperscanners 1 mit der Person 2. Mit Hilfe des Körperscanners 1 können 3D Körpermodelle, insbesondere eine äußere Form bzw. eine Silhouette, der Person 2 erstellt werden. Das Körpermodell kann beispielsweise in der Bekleidungsbranche verwendet werden, um Kleidungsstücke anpassen oder passend finden zu können. Weiterhin können die Körpermodelle auch für medizinische Zwecke, zur Dokumentation sportlichen Fortschritten, zur Erstellung von Computermodellen der Perons usw. verwendet werden.

Der Körperscanner 1 umfasst ferner eine Scannereinheit 3, die zum Erfassen der Person zumindest einen Tiefensensor 4 aufweist. Mit Hilfe des Tiefensensors 4 kann die Person 2 in einem Sichtfeld 5 erfasst werden. Der Tiefensensor 4 erfasst Tiefendaten des Sichtfelds 5, anhand derer das Körpermodell ermittelt wird. Der zumindest eine Tiefensensor 4 erfasst sozusagen 3D-Daten des Sichtfeldes 5.

Des Weiteren umfasst der Körperscanner 1 eine Plattform 6 zur Aufnahme der Person 2. Auf der Plattform 6 kann die Person 2 stehen, wobei mittels einer Platzierung der Plattform 6 ein Ort der Person 2 vorgegeben ist.

Die Plattform 6 kann beispielsweise als Drehteller ausgebildet sein, so dass die Person 2 gedreht werden kann, um die Person 2 von allen Seiten erfassen zu können. Die Plattform 6 kann dazu einen Elektromotor umfassen. Zusätzlich oder alternativ kann die Plattform 6 auch eine Wiegeeinheit umfassen, mittels der ein Gewicht der Person 2 erfasst werden kann. Zusätzlich oder alternativ kann die Plattform 6 einen Sensor zur Erfassung von Körperparametern aufweisen. Neben der besagten Wiegeeinheit kann die Plattform 6 zusätzlich oder alternativ auch einen Körperfettsensor, einen Pulssensor usw. aufweisen.

Des Weiteren kann, wie hier gezeigt ist, die Scannereinheit 3 ein Fußelement 7 aufweisen, um die Scannereinheit 3 auf einem Untergrund abzustellen.

Des Weiteren weist die Scannereinheit 3 des vorliegenden Ausführungsbeispiels einen Spiegel 8 auf, mit dessen Hilfe sich die Person 2 selbst betrachten kann.

Außerdem umfasst der Körperscanner 1 gemäß dem vorliegenden Ausführungsbeispiel eine Steuereinheit 9. In diesem Ausführungsbeispiel weist die Scannereinheit 3 die Steuereinheit 9 auf. Mit Hilfe der Steuereinheit 9 kann der Körperscanner 1 gesteuert werden. Beispielsweise kann die Steuereinheit 9 die mittels dem Tiefensensor 4 erfassten Daten auswerten und das Körpermodell erzeugen. Die Steuereinheit 9 kann zusätzlich oder alternativ die Plattform 6 steuern.

Der 3D Körperscanner 1 umfasst ferner einen hier nicht gezeigten Energieanschluss, um den 3D Körperscanner 1 mit elektrischer Energie, beispielsweise aus einem Stromnetz, zu versorgen. Beispielsweise weist die Scannereinheit 3 den Energieanschluss auf.

Gemäß dem vorliegenden Ausführungsbeispiel weist die Plattform 6 einen Energiespeicher 11 auf, um die Plattform 6 mit Energie versorgen zu können. Der Energiespeicher 11 kann wiederaufladbar sein. Beispielsweise umfasst der Energiespeicher 11 einen Akkumulator.

Gemäß dem vorliegenden Ausführungsbeispiel weist die Scannereinheit 3 und die Plattform 6 jeweils eine Funkschnittstelle 17a, 17b auf, so dass beide kabellos miteinander kommunizieren und/oder Daten austauschen können. Die beiden Funkschnittstellen 17a, 17b können beispielsweise als Bluetooth ausgebildet sein.

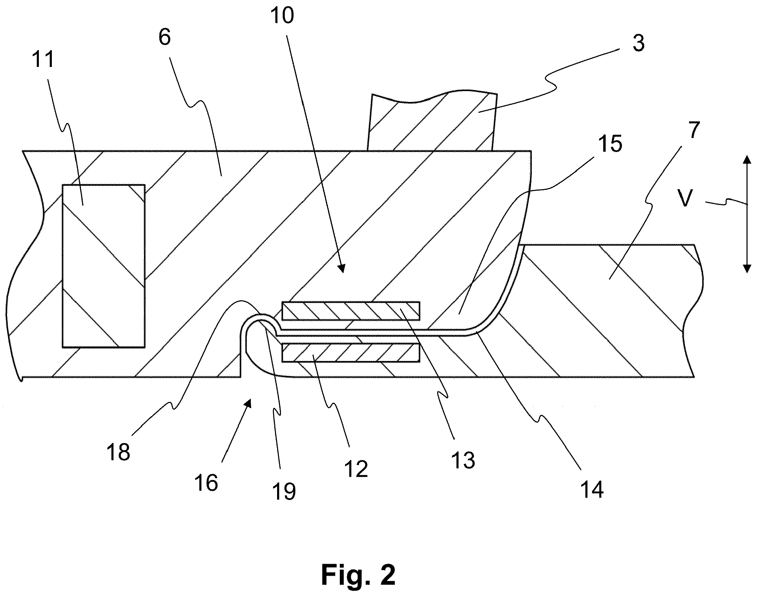

Figur 2 zeigt eine schematische Schnittansicht der an die Scannereinheit 3 angekoppelten Plattform 6 mit einer Energieübertragungsanordnung 10.

Mittels der Energieübertragungsanordnung 10 kann elektrische Energie kontaktlos zwischen der Scannereinheit 3 und der Plattform 6 ausgetauscht werden. Dadurch kann der Körperscanner 1 auf einfache Weise ausgebildet werden. Außerdem kann die Plattform 6 zur Aufnahme der Person 2 flexibler positioniert werden.

Mit Hilfe der Energieübertragungsanordnung 10 kann ferner der Energiespeicher 11 geladen werden, um die Plattform 11 autark betreiben zu können. Der Energiespeicher 11 kann beispielsweise einen Akkumulator umfassen.

Gemäß dem vorliegenden Ausführungsbeispiel überträgt die Energieübertragungsanordnung 10 die elektrische Energie, wenn die Plattform 6 an die Scannereinheit 3 angekoppelt ist. Des Weiteren weist der Körperscanner 1 eine Koppelanordnung 16 auf, um die Plattform 6 mit der Scannereinheit 3 zu koppeln. Dadurch kann sichergestellt werden, dass beim Übertragen der Energie die Plattform 6 mit der Scannereinheit 3 gekoppelt bleibt. Die Koppelanordnung 16 umfasst gemäß dem vorliegenden Ausführungsbeispiel eine Koppelaussparung 18, in die ein Koppelelement 19 eingreift. Die Koppelanordnung 16 kann die Plattform 6 beispielsweise formschlüssig mit der Scannereinheit 3 koppeln. Zusätzlich oder alternativ kann die Koppelanordnung 16 die Plattform 6 auch kraftschlüssig mit der Scannereinheit 3 koppeln.

Weiterhin umfasst die Energieübertragungsanordnung 10 gemäß dem vorliegenden Ausführungsbeispiel eine Sendeeinheit 12 und eine Empfangseinheit 13. Mit Hilfe der Sendeeinheit 12 kann elektrische Energie gesendet und mit der Empfangseinheit 13 kann die elektrische Energie empfangen werden. Gemäß dem vorliegenden Ausführungsbeispiel weist die Scannereinheit 3 die Sendeeinheit 12 und die Plattform 6 die Empfangseinheit 13 auf. Zusätzlich oder alternativ kann auch die Scannereinheit 3 eine Empfangseinheit 13 und die Plattform 6 eine Sendeeinheit 12 aufweisen.

Die Sende- und die Empfangseinheit 12, 13 können beispielsweise als Sende- und Empfangsspule ausgebildet sein, so dass die elektrische Energie induktiv übertragen wird. Zusätzlich oder alternativ kann die Energieübertragungsanordnung 10 auch derart ausgebildet sein, dass die elektrische Energie kapazitiv übertragen wird. Dann können die Sende- und die Empfangseinheit 12, 13 entsprechende Kondensatorelemente sein.

Des Weiteren weist die Scannereinheit 3, insbesondere das Fußelement 7, eine Aussparung 14 auf, in die die Plattform 6 mit einem Einführabschnitt 15 eingeführt werden kann. Dadurch kann sichergestellt werden, dass die Energieübertragungsanordnung 10 die elektrische Energie übertragen kann. Insbesondere wird sichergestellt, dass die Sende- und die Empfangseinheit 12, 13 zueinander korrekt angeordnet sind.

Außerdem wird die Plattform 6 aus der Richtung an die Scannereinheit 3 herangeführt, an welcher der in Figur 1 gezeigte Spiegel 8 angeordnet ist. Die Plattform 6 wird somit aus der Richtung an die Scannereinheit 3 herangeführt bzw. angekoppelt, an der der Tiefensensor 4 angeordnet ist. Die Plattform 6 wird somit von vorne an die Scannereinheit 3 herangeführt bzw. angekoppelt.

Gemäß dem vorliegenden Ausführungsbeispiel sind die Sende- und die Empfangseinheit 12, 13 übereinander angeordnet. D.h. die Sende- und die Empfangseinheit 12, 13 sind in einer Vertikalrichtung V voneinander beabstandet angeordnet.

Die vorliegende Erfindung ist nicht auf die dargestellten und beschriebenen Ausführungsbeispiele beschränkt. Abwandlungen im Rahmen der Patentansprüche sind ebenso möglich wie eine Kombination der Merkmale, auch wenn diese in unterschiedlichen Ausführungsbeispielen dargestellt und beschrieben sind.

### Bezugszeichenliste

- 1: 3D Körperscanner
- 2: Person
- 3: Scannereinheit
- 4: Tiefensensor
- 5: Sichtfeld
- 6: Plattform
- 7: Fußelement
- 8: Spiegel
- 9: Steuereinheit
- 10: Energieübertragungsanordnung
- 11: Energiespeicher
- 12: Sendeeinheit
- 13: Empfangseinheit
- 14: Aussparung
- 15: Einführabschnitt
- 16: Koppelanordnung
- 17: Funkschnittstelle
- 18: Koppelaussparung
- 19: Koppelelement

- V: Vertikalrichtung

## Patentansprüche

1. 3D Körperscanner (1) zum Erstellen von 3D Körpermodellen, insbesondere einer äußeren Körperform, einer Person (2)
mit einer Scannereinheit (3), die zumindest einen Tiefensensor (4) zur räumlichen Erfassung eines Sichtfeldes (5) umfasst, und
mit einer Plattform (6) zur Aufnahme der Person (2),
**dadurch gekennzeichnet,**
**dass** der 3D Körperscanner (1) eine Energieübertragungsanordnung (10) umfasst, mittels der elektrische Energie kontaktlos zwischen der Scannereinheit (3) und der Plattform (6) übertragen werden kann.

2. 3D Körperscanner (1) nach dem vorherigen Anspruch, **dadurch gekennzeichnet, dass** die Plattform (6) einen elektrischen Energiespeicher (11) umfasst.

3. 3D Körperscanner (1) nach einem oder mehreren der vorherigen Ansprüche, **dadurch gekennzeichnet, dass** die Energieübertragungsanordnung (10) eine Sendeeinheit (12) zum Senden und eine Empfangseinheit (13) zum Empfangen der elektrischen Energie umfasst, wobei vorzugsweise die Scannereinheit (3) die Sendeeinheit (12) und die Plattform (6) die Empfangseinheit (13) umfasst.

4. 3D Körperscanner (1) nach einem oder mehreren der vorherigen Ansprüche, **dadurch gekennzeichnet, dass** die Scannereinheit (3) ein Fußelement (7) aufweist, welches vorzugsweise die Sendeeinheit (12) umfasst.

5. 3D Körperscanner (1) nach einem oder mehreren der vorherigen Ansprüche, **dadurch gekennzeichnet, dass** die Scannereinheit (3), insbesondere das Fußelement (7), eine Aussparung (14) aufweist und dass die Plattform (6) einen zur Aussparung (14) korrespondierenden Einführabschnitt (15) aufweist, so dass die Plattform (6) mittels dem Einführabschnitt (15) in der Aussparung (14) angeordnet werden kann.

6. 3D Körperscanner (1) nach einem oder mehreren der vorherigen Ansprüche, **dadurch gekennzeichnet, dass** die Scannereinheit (3), insbesondere das Fußelement (7), im Bereich der Aussparung (14) die Sendeeinheit (12) aufweist und die Plattform (6) im Einführabschnitt (15) die Empfangseinheit (13) aufweist oder
dass die Scannereinheit (3), insbesondere das Fußelement (7), im Bereich der Aussparung (14) die Empfangseinheit (13) aufweist und die Plattform (6) im Einführabschnitt (15) die Sendeeinheit (12) aufweist.

7. 3D Körperscanner (1) nach einem oder mehreren der vorherigen Ansprüche, **dadurch gekennzeichnet, dass** die Scannereinheit (3) und/oder die Plattform (6) eine Koppelanordnung (16) aufweist, so dass die Plattform (6) zur Energieübertragung an die Scannereinheit (3) angekoppelt werden kann.

8. 3D Körperscanner (1) nach einem oder mehreren der vorherigen Ansprüche, **dadurch gekennzeichnet, dass** zur Energieübertragung die Sendeeinheit (12) und die Empfangseinheit (13) deckungsgleich in einer Horizontalrichtung der Scannereinheit (3) übereinander angeordnet werden können.

9. 3D Körperscanner (1) nach einem oder mehreren der vorherigen Ansprüche, **dadurch gekennzeichnet, dass** bei vorgesehener Platzierung des 3D Körperscanners (1) die Sendeeinheit (12) unter der Empfangseinheit (13) angeordnet ist.

10. 3D Körperscanner (1) nach einem oder mehreren der vorherigen Ansprüche, **dadurch gekennzeichnet, dass** die Energieübertragungsanordnung (10) derart ausgebildet ist, dass die elektrische Energie induktiv und/oder kapazitiv übertragen werden kann.

11. 3D Körperscanner (1) nach einem oder mehreren der vorherigen Ansprüche, **dadurch gekennzeichnet, dass** die Sendeeinheit (12) eine Sendespule und die Empfangseinheit (13) eine Empfangsspule umfasst, wobei zur Energieübertragung die Sendespule und die Empfangsspule koaxial zueinander angeordnet sind.

12. 3D Körperscanner (1) nach einem oder mehreren der vorherigen Ansprüche, **dadurch gekennzeichnet, dass** der 3D Körperscanner (1) zumindest einen 3D-Sensor umfasst.

13. 3D Körperscanner (1) nach einem oder mehreren der vorherigen Ansprüche, **dadurch gekennzeichnet, dass** die Plattform (6) eine Wiegeeinheit aufweist und/oder als Drehteller ausgebildet ist.

14. 3D Körperscanner (1) nach einem oder mehreren der vorherigen Ansprüche, **dadurch gekennzeichnet, dass** die Scannereinheit (3) und die Plattform (6) jeweils eine Funkschnittstelle (17a, 17b) aufweisen, so dass zwischen der Scannereinheit (3) und der Plattform (6) eine drahtlose Funkverbindung ausgebildet werden kann.

15. 3D Körperscanner (1) nach einem oder mehreren der vorherigen Ansprüche, **dadurch gekennzeichnet, dass** die Scannereinheit (3) einen Spiegel (8) umfasst.
